# FASCICULE DE BREVET EUROPEEN

(11) **EP 0 608 380 B1**
(45) Date de publication et mention de la délivrance du brevet: **20.09.1995**
(21) Numéro de dépôt: 92923833.5
(22) Date de dépôt: 14.10.1992
(51) Int. Cl.: G01N 3/08, G01N 3/18

(54) **DISPOSITIF DE COMPRESSION D'ECHANTILLON A TEMPERATURE ELEVEE NOTAMMENT POUR MATERIAUX AVANCES A RESISTANCE MECANIQUE**
VORRICHTUNG ZUM HOCHTEMPERATURVERDICHTEN VON PROBEN AUS HOCHFESTEN MATERIALEN
DEVICE FOR COMPRESSING A SPECIMEN AT HIGH TEMPERATURE, IN PARTICULAR FOR ADVANCED TENSILE MATERIALS

(30) Priorité: 15.10.1991 FR 9112686
(43) Date de publication de la demande: 03.08.1994
(73) Titulaire: COMMISSARIAT A L'ENERGIE ATOMIQUE, 75015 Paris (FR)
(72) Inventeur: AZZOLINI, Raymond, F-91180 S.-Germain-les-Arpajon (FR); ROCHARD, Pierre, F-78120 Orphin (FR)
(74) Mandataire: Ilgart, Jean-Christophe
(86) Numéro de dépôt international: FR9200968
(87) Numéro de publication internationale: WO9308456

(56) Documents cités:
- EP-A- 0 237 098
- FR-A- 2 398 297
- US-A- 2 346 281

## Description

L'invention a pour objet un dispositif de compression qui garantit une compression pure d'un échantillon ayant deux faces parallèles entre lesquelles il est comprimé sans qu'aucune opération de réglage préalable de la presse soit nécessaire même à haute température et pour de fortes charges.

Les machines d'essais mécaniques classiques utilisées pour ce genre d'essais nécessitent normalement des réglages délicats pour obtenir une sollicitation uniaxiale rigoureuse, c'est-à-dire une compression pure et uniforme sur l'échantillon. Elles sont essentiellement composées en général de deux parties qui se font face (par des faces planes) et dont l'une est un support fixe et l'autre un piston mobile. L'échantillon est comprimé par l'avance du piston, mais des opérations longues et difficiles sont nécessaires pour rétablir un alignement correct des deux parties.

Le document EP-A-0 237 098 décrit un dispositif de compression d'une pile de feuilles juxtaposées dans lequel la pile est comprimée entre les faces planes de deux blocs dont l'autre extrémité pour chacun est convexe. Ces extrémités sont en contact direct avec deux autres faces planes et elles peuvent s'incliner pour ajuster tout jeu éventuel pouvant apparaître dans l'alignement, lors de l'essai de compression.

Le document FR-A-2 398 297 décrit un appareil triaxial rotulé comprenant deux têtes rotulées dans lesquelles sont incorporés des moyens destinés à avoir un centre de rotation à la surface de l'échantillon, ces moyens étant des contacts du type sphère-sphère ayant le même centre de courbure à la surface de l'échantillon.

Une solution satisfaisante à ce problème est offerte par l'invention, qui se caractérise en ce que le support et le piston sont chacun composés de deux parties réunies par une rotule respective, les parties qui portent les faces planes étant constituées de façon à coulisser l'une dans l'autre.

Ces parties forment donc un ensemble qu'on peut considérer comme monobloc sauf dans la direction de compression de l'éprouvette. La double liaison par une rotule permet de ne transmettre, en combinaison avec l'ajustage à coulisse, que des efforts de compression malgré toutes les déformations que la chaleur peut occasionner sur l'appareil.

L'indépendance du dispositif par rapport à la machine d'essais mécaniques offre d'autres avantages. Il est facile d'insérer le dispositif qui porte les faces planes dans une enceinte de chauffage. Ces parties et l'échantillon sont alors chauffés à la température d'essai souhaitée alors que les autres parties qui transmettent l'effort de compression restent relativement froides.

Le dispositif de compression est réalisé en graphite à faible taux de cendres (pour éviter les dégazages à hautes températures), car ce matériau présente de bonnes qualités mécaniques qui s'améliorent à température élevée. Les faces planes peuvent cependant avantageusement appartenir à des enclumes en céramique, en zircone par exemple, pour assurer une bonne tenue mécanique à haute température.

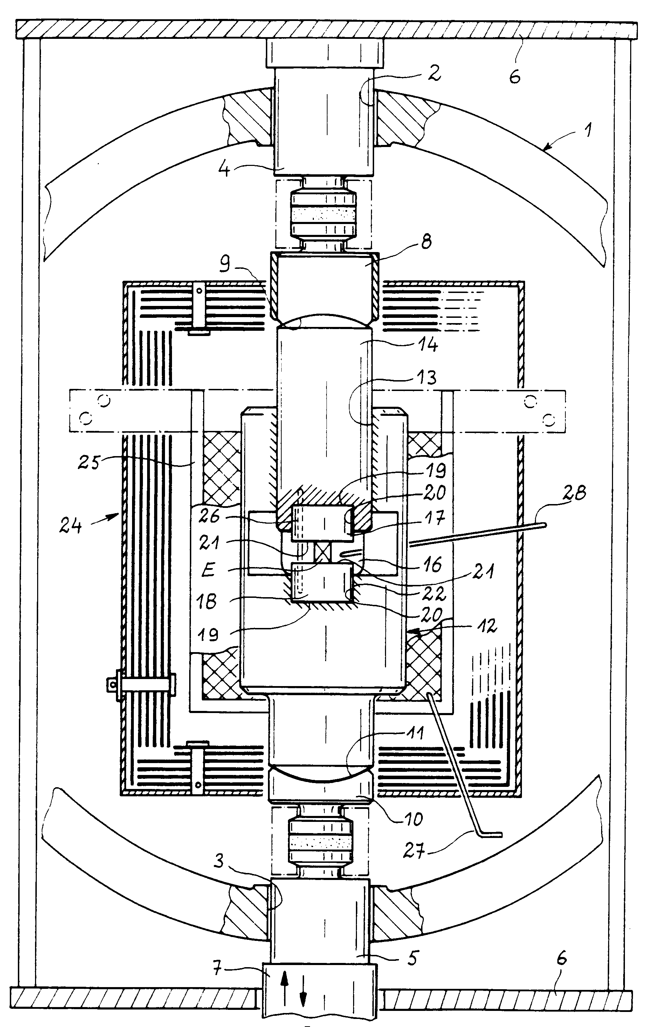

La figure unique permettra de décrire à titre purement illustratif une réalisation de l'invention.

La machine d'essais comprend une enceinte de four 1 formée de deux demi-coquilles cylindriques se refermant l'une sur l'autre et dont une seule est représentée ici. Cette demi-coquille est percée de deux ouvertures 2 et 3 diamétralement opposées : une tige de piston 4 fixée à un bâti 6 immobile passe à travers l'une et une tige fixe de support 5 par l'autre. Celle-ci est fixée à un vérin 7 mobile asservi par un système électronique de commande pour comprimer un échantillon E. Le piston 4 s'étend jusqu'à un embout 8 dont la face d'extrémité libre est en forme de calotte sphérique concave 9. La tige de support 5 a aussi un embout 10 terminé par une face d'extrémité libre en forme de calotte sphérique concave 11.

Le support du dispositif de compression 12 est posé sans aucun réglage préalable sur la calotte sphérique concave 11 par une surface en forme de calotte sphérique convexe qui forme donc une liaison à rotule avec la calotte sphérique 11. Il porte à son autre extrémité un alésage 13 à section circulaire dans lequel coulisse une extrémité de piston 14 du dispositif de compression, de même section et dont l'extrémité supérieure est terminée par une calotte sphérique convexe qui s'ajuste partiellement dans la calotte sphérique concave 9 de manière à former une autre liaison à rotule. II n'existe qu'un très faible jeu entre l'extrémité du piston 14 et l'alésage 13: une erreur d'alignement entre le piston 4 et la tige fixe de support 5 se traduit donc par une position différente du support 12 et de l'extrémité de piston 14, mais la force transmise entre ces deux pièces reste parallèle à l'axe de l'extrémité du piston 14.

Le support d'échantillon 12 est évidé selon une excavation transversale 16 dans laquelle l'alésage 13 débouche. On y introduit l'échantillon E. Il est en réalité comprimé entre deux enclumes de zircone 17 et 18 qui font respectivement partie de l'extrémité de piston 14 et du support 12. Chacune des enclumes 17 et 18 est plus précisément un cylindre court dont une face plane arrière 19 est appuyée sur la surface de fond, également plane, d'un trou borgne 20 de même section évidé à l'extrémité de l'extrémité de piston 14 et dans le support d'échantillon 12, et une face plane avant 21 sert à appuyer l'échantillon E et à le comprimer entre ces deux faces avant 21 qui sont parallèles ; enfin, une surface latérale circulaire 22 des enclumes 17 et 18 et ajustée dans la paroi latérale des trous borgnes 20.

Le dispositif de chauffage est composé d'un bouclier d'isolation thermique 24 et de deux demi-résistors 25 assurant le chauffage. Le bouclier d'isolation thermique 24 permet de confiner le dispositif de compression dans une zone de chauffe composée par deux demi-résistors 25, l'un et l'autre reliés aux deux demi-coquilles de L'enceinte du four 1. La configuration adoptée permet de porter le dispositif de compression (piston 14, support 12, et enclumes 17 et 18) à la même température que l'échantillon E, alors que la tige de support S et le piston 4 restent à une température presque invariable. Le dispositif de chauffage est pourvu d'un thermocouple 27 permettant la régulation de la température, et d'un thermocouple 28 assurant la mesure de la température sur l'échantillon E.

Le dispositif de compression permet la mesure des déformations de l'échantillon pendant la durée totale d'un essai. Sa forme (excavation transversale 16 du support 12 et forme des enclumes 17 et 18) adaptée à une extensométrie sans contact au moyen d'un laser permet la mesure des grandeurs suivantes du matériau testé à haute température (module d'Young, coefficient de dilatation, déformations élastiques et plastiques). La mesure se fait par une fente 26 réalisée dans le bouclier thermique 24.

Le dispositif de compression réalisé est apte à solliciter des échantillons en céramique jusqu'à 2200°C sous vide ou sous atmosphère contrôlée, et des produits carbonés jusqu'à 3000°C sous atmosphère contrôlée. Les charges pouvant être atteintes sont de 100 000 N à 1500°C par exemple.

## Revendications

1. Dispositif de compression d'échantillon (E) composée d'un support fixe (5, 12) de l'échantillon et d'un piston mobile (4, 14) de compression de l'échantillon (E), le support et le piston étant pourvus de faces planes (21) entre lesquelles l'échantillon (E) est appuyé, caractérisé en ce que le support et le piston sont chacun composés de deux parties réunies par une rotule respective (9, 11), les parties (12, 14) qui portent les faces planes (21) étant constituées de façon à coulisser l'une dans l'autre.

2. Dispositif de compression d'échantillon suivant la revendication 1, caractérisé par une enceinte de chauffage (24) qui enveloppe les parties (12, 14) du support et du piston qui portent les faces planes.

3. Dispositif de compression d'échantillon suivant la revendication 1 ou 2, caractérisé en ce que les parties (12, 14) du support et du piston qui portent les faces planes sont en graphite.

4. Dispositif de compression d'échantillon suivant la revendication 3, caractérisé en ce que les parties du support qui portent les faces planes sont pourvues d'enclumes (17, 18) auxquelles appartiennent les faces planes (21).

5. Dispositif de compression d'échantillon suivant la revendication 4, caractérisé en ce que les enclumes (17, 18) sont en zircone.

## Patentansprüche

1. Vorrichtung zum Verdichten von Proben (E), gebildet durch einen festen Probenträger (5, 12) und einen beweglichen Verdichtungskolben (4, 14) der Probe (E), wobei der Träger und der Kolben mit Planflächen (21) versehen sind, zwischen denen die Probe (E) gepreßt wird,
**dadurch gekennzeichnet**,
daß der Träger und der Kolben jeweils gebildet werden durch zwei durch ein Kugelgelenk (9, 11) verbundene Teile, wobei die Teile (12, 14), die die Planflächen (21) tragen, so ausgebildet sind, daß sie ineinander gleiten.

2. Probenverdichtungsvorrichtung nach Anspruch 1, gekennzeichnet durch einen Heizbehälter (24), der die Teile (12, 14) des Trägers und des Kolbens umhüllt, die die Planflächen tragen.

3. Probenverdichtungsvorrichtung nach Anspruch 1 oder 2, dadurch gekennzeichnet, daß die Teile (12, 14) des Trägers und des Kolbens, die die Planflächen tragen, aus Graphit sind.

4. Probenverdichtungsvorrichtung nach Anspruch 3, dadurch gekennzeichnet, daß die Teile des Trägers, die die Planflächen tragen, mit Ambossen (17, 18) versehen sind, zu denen die Planflächen (21) gehören.

5. Probenverdichtungsvorrichtung nach Anspruch 4, dadurch gekennzeichnet, daß die Ambosse (17, 18) aus Zirkon sind.

## Claims

1. Device for compressing a specimen (E) constituted by a fixed support (5, 12) for the specimen and a mobile piston (4, 14) for compressing the specimen (E), the support and the piston having planar faces (21) between which bears the specimen (E), characterized in that the support and the piston are in each case constituted by two parts joined by a respective swivel joint (9, 11), the parts (12, 14) carrying the planar faces (21) being constructed so as to slide in one another.

2. Specimen compression device according to claim 1, characterized by a heating enclosure (24), which envelopes the parts (12, 14) of the support and the piston which carry the planar faces.

3. Specimen compression device according to claim 1 or 2, characterized in that the parts (12, 14) of the support and the piston carrying the planar faces are made from graphite.

4. Specimen compression device according to claim 3, characterized in that the parts of the support carrying the planar faces are provided with anvils (17, 18) to which belong the planar faces (21).

5. Specimen compression device according to claim 4, characterized in that the anvils (17, 18) are made from zirconia.
